# EUROPÄISCHE PATENTANMELDUNG

(11) **EP 1 293 217 A1**
(43) Veröffentlichungstag der Anmeldung: **19.03.2003**
(21) Anmeldenummer: 02400042.4
(22) Anmeldetag: 15.09.2002
(51) Int. Cl.: A61L 17/00

(54) **Medizinisches Nahtmaterial mit kovalent angebundenem pharmazeutischen Wirkstoff**

(30) Priorität: 18.09.2001 DE 10146814
(71) Anmelder: UroNova GmbH, 91058 Erlangen (DE)
(72) Erfinder: Hildebrandt, Peter, 90482 Nürnberg (DE); von der Assen, Nikolaus, 49439 Steinfeld (DE)

(57) **Zusammenfassung**

Ein medizinisches Nahtmaterial ist mit einem vorzugsweise im wesentlichen zylindrischen Träger (1) versehen. An der Trägeroberfläche (2) ist ein pharmazeutischer Wirkstoff (3) über eine kovalente Bindung (4) dauerhaft gebunden.

## Beschreibung

Die Erfindung betrifft ein medizinisches Nahtmaterial. Solches Nahtmaterial wird üblicherweise verwendet, um biologisches Gewebe zusammenzubringen und zu fixieren, bis eine Heilung der zusammengeführten Gewebe erfolgt ist. In der Gefäßchirurgie wird Nahtmaterial auch verwandt, um Blutgefäße durch Naht zu vereinigen (Fachjargon: zu anastomosieren).

Es werden resorbierbare und nicht resorbierbare Nahtmaterialien unterschieden. Resorbierbare Nahtmaterialien brauchen nicht entfernt zu werden, da sie nach einer bestimmten Zeit durch die Körperflüssigkeiten aufgelöst werden. Sie werden hauptsächlich in der inneren Medizin verwandt. Gängige Materialien sind hier Polyglykolsäuren und Polydioxanone. Nicht resorbierbare Materialien dagegen müssen entfernt werden und finden daher bei der Versorgung äußerer Wunden Verwendung, aber auch in Fällen, in denen eine dauerhafte Zugfestigkeit des Nahtmaterials erforderlich ist, wie im Bereich des schlagenden Herzens. Gängige Materialien sind hier Polyester und Polyamide. Weiterhin werden monofile und geflochtene Nahtmaterialien unterschieden.

Als schwerwiegendes Problem tritt bei allen Nahtmaterialien häufig die Besiedlung mit Bakterien ein. Damit wird das Nahtmaterial zum Infektionsherd und eine Behandlung der ohnehin oft geschwächten Patienten mit Antibiotika und den damit verbundenen Nebenwirkungen wird notwendig. Studien haben gezeigt, daß geflochtene Nahtmaterialien bis zu zehnfach höhere bakterielle Besiedlung aufweisen als monofile Nahtmaterialien. Jedoch auch monofile Nahtmaterialien sind nicht frei von bakterieller Besiedlung und weisen zudem Nachteile in der Handhabung, wie z.B. der Knotbarkeit auf.

Ein weiteres Problem stellt übermäßiges Zellwachstum (Fachjargon: Proliferation) als Reaktion des Gewebes auf die Verletzung durch die Naht dar. In Blutgefäßen kann dies besonders negative Auswirkungen haben, da durch die wuchernden Zellen deren Durchmesser verengt oder gar vollständig verschlossen wird.

Ein Ansatz aus dem Stand der Technik zur Lösung der Infektionsproblematik ist der US 3 896 813 zu entnehmen. In dieser Druckschrift ist ein Nahtmaterial offenbart, das mit wasserunlöslichen bioziden Salzen imprägniert ist. Die bioziden Salze sind in das in das geflochtene Nahtmaterial inkorporiert, was eine langanhaltende Wirkstoffquelle zur Freigabe in den Körper schaffen soll.

Der dem Stand der Technik entnommene Ansatz verfolgt eine Herstellung antimikrobieller Eigenschaften durch die Imprägnierung mit bioziden Salzen. Diese soll durch das Eintauchen des geflochtenen Fadenmaterials in Lösungen der entsprechenden Salze und anschließendes Trocknen erfolgen. Da die Stoffe dabei nicht chemisch an die Oberfläche des Nahtmaterials gebunden werden, erfolgt eine langsame Wirkstofffreigabe. Ist das Reservoir an Wirkstoffen aufgebraucht, so nimmt das Nahtmaterial wieder seine ursprünglichen Eigenschaften bezüglich der Anfälligkeit für bakterielle Besiedlung an. Weiterhin kann die Freigabe der Wirkstoffe unerwünschte Nebenwirkungen, Unverträglichkeitsreaktionen oder Wechselwirkungen mit anderen Medikamenten die der Patient erhält verursachen. Damit ist die mit diesem Stand der Technik erreichbare Wirkung nach wie vor verbesserungsbedürftig.

Ein Ansatz aus dem Stand der Technik zur Lösung der Problematik des übermäßigen Zellwachstums ist der WO 98/47432 zu entnehmen. In dieser Druckschrift ist ein Nahtmaterial offenbart, das ein radioaktives β-strahlendes Element enthält. Durch die Einwirkung der β-Strahlung auf die Zellen soll deren übermäßiges Wachstum verhindert werden.

Der dem Stand der Technik entnommene Ansatz verfolgt eine Herstellung antiproliferativer Eigenschaften durch die Verwendung β-strahlender Elemente. Die therapeutische Anwendung radioaktiver Stoffe verursacht jedoch auch grundsätzlich Zellschädigungen unerwünschter Art. Weiterhin ist bei einem radioaktiven Nahtmaterial von einem erheblichen Mehraufwand an Sicherheitsmaßnahmen bei Herstellung, Transport und Lagerung auszugehen. Damit ist dieser Stand der Technik nach wie vor verbesserungsbedürftig.

Eigene Versuchsreihen konnten zeigen, daß für die Bekämpfung der erläuterten Probleme bei der Anwendung von medizinischem Nahtmaterial weder eine Wirkstofffreigabe mit den unerwünschten Nebenwirkungen, noch die Verwendung β-strahlender Elemente unter Inkaufnahme deren Gefahrenpotentials notwendig ist. Es wurde nachgewiesen, daß bereits der Kontakt des umliegenden Gewebes mit an das Nahtmaterial fest gebundenen pharmazeutischen Wirkstoffen einen vergleichbaren Effekt hat wie es bei ins Gewebe freigegebenen Wirkstoffen der Fall ist. Durch die Verwendung fest an die Oberfläche gebundener antimikrobieller pharmazeutischer Wirkstoffe kann eine bakterielle Besiedlung des Nahtmaterials verhindert werden, ohne die mit der Wirkstofffreigabe verbundenen Nachteile und Nebenwirkungen in Kauf nehmen zu müssen. Antiproliferative Eigenschaften lassen sich ohne unerwünschte Zellschädigung erreichen, indem pharmazeutische Zytostatika anstelle radioaktiver Elemente verwendet werden. Bei Anwendungen, die eine Beschleunigung des Zellwachstums erfordern, ist auch eine dauerhafte Anbindung von zellwachstumsfördernden Stoffen vorstellbar.

Vor diesem Hintergrund schlägt nun die Erfindung laut Kennzeichnungsteil des Anspruches 1 zur Lösung der eingangs geschilderten Problematik ein medizinisches Nahtmaterial vor, an dessen Oberfläche ein pharmazeutischer Wirkstoff über eine kovalente Bindung dauerhaft gebunden ist. Durch die kovalente Bindung, die chemisch stabilste Bindungsform, erfolgt keine Wirkstofffreigabe und die erwünschten Eigenschaften der Beschichtung haben Bestand. Das Auftreten von unerwünschten Nebenwirkungen, Unverträglichkeitsreaktionen oder Wechselwirkungen durch eine Freigabe des verwendeten Wirkstoffes ist auszuschließen. So kann beispielsweise durch die kovalente Anbindung von antimikrobiellen Stoffen der bakteriellen Besiedlung, durch die kovalente Anbindung von Zytostatika dem übermäßigen Zellwachstum entgegengewirkt werden.

Gemäß bevorzugten Ausführungsformen kann das Grundmaterial aus einem Polyamid, einem Polyester oder Polypropylen bestehen. Auch die Verwendung von resorbierbaren Materialien wie Polyglkolsäuren oder Polydioxanonen ist möglich. Weiterhin ist eine monofile oder geflochtene Ausbildung anwendbar.

Die kovalente Anbindung erfolgt vorzugsweise über ein Zwischenmolekül (Fachjargon: Spacer). Die Auswahl von angepaßten Zwischenmolekülen ermöglicht die kovalente Bindung verschiedener Wirkstoffe an verschiedene Grundmaterialien. So kann beispielsweise die antimikrobielle Hyaluronsäure über einen Ethyleniminspacer an das Grundmaterial aus Polypropylen gebunden werden.

Zusammenfassend haben experimentelle Untersuchungen mit erfindungsgemäß ausgerüstetem Nahtmaterial, bei dem Hyaluronsäure auf Polypropylen eingesetzt wird, eine signifikante Verringerung der Bakterienbesiedlung und des Zellwachstums ergeben. So konnte bei in vitro Versuchen durch Eintauchen entsprechender Proben in Bakteriensuspensionen mit Staphylococcus epidermidis über mehrere Tage eine Reduktion der Bakterienbesiedlung auf ein Zehntel bis hin z.T. auf Null nachgewiesen werden. Die Kultivierung proliferativer Mäusezellen auf dem gleichen erfindungsgemäß ausgerüsteten Nahtmaterial zeigte eine signifikante Verlangsamung des Zellwachstums. So konnte im Falle der Hyaluronsäure sogar mit nur einem Wirkstoff sowohl eine antimikrobielle, als auch eine zytostatische Wirkung erzielt werden.

Die beigefügten Zeichnungen erläutern die Merkmale, Einzelheiten und Vorteile des Erfindungsgegenstandes näher. Es zeigen:
- Fig. 1: einen Querschnitt durch ein monofiles Nahtmaterial mit ausschnittweiser Vergrößerung der Oberfläche
- Fig. 2: einen Querschnitt durch ein geflochtenes Nahtmaterial mit ausschnittweiser Vergrößerung der Oberfläche

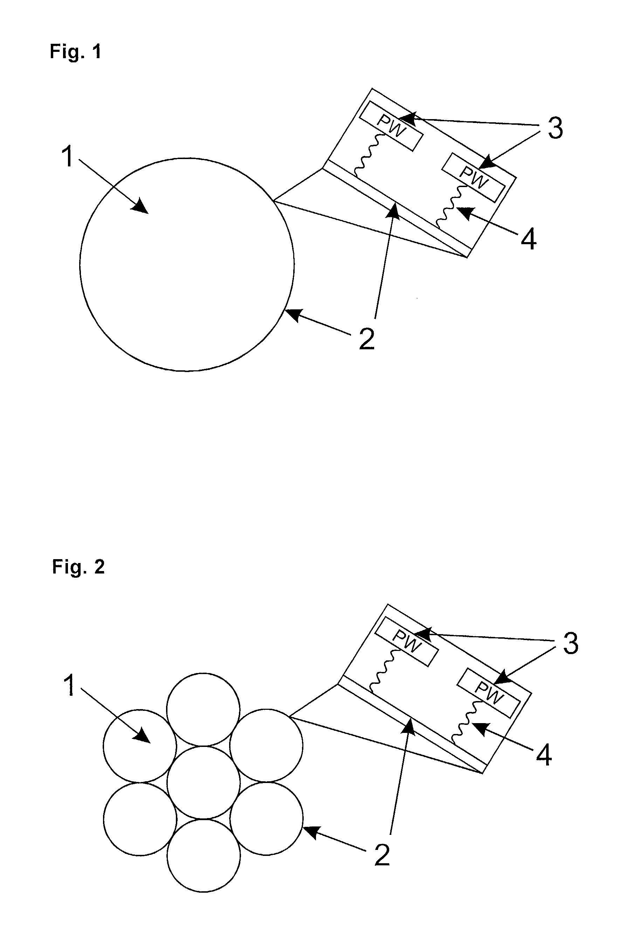

Fig. 1 zeigt einen monofil ausgestalteten Träger 1 für ein medizinisches Nahtmaterial, das beispielsweise aus Polypropylen besteht. An dessen Oberfläche 2 ist ein pharmazeutischer Wirkstoff 3 über eine kovalente Bindung 4 gebunden.

Fig. 2 zeigt einen geflochten ausgestalteten Träger 1 für ein medizinisches Nahtmaterial, das beispielsweise aus Polyester besteht. An dessen Oberfläche 2 ist ein pharmazeutischer Wirkstoff 3 über eine kovalente Bindung 4 gebunden.

Nachfolgend wird in Form einer Auflistung einzelner Verfahrensschritte beispielhaft ein Beschichtungsverfahren von Hyaluronsäure auf einem Grundmaterial aus Polypropylen (PP) wiedergegeben:
- PP-Nahtmaterial 2 Minuten in 500ml konzentrierte Schwefelsäure + 1g KMnO₄ einlegen.
- PP-Nahtmaterial drei mal in deionisiertem Wasser waschen.
- PP-Nahtmaterial 5 Minuten in 500ml Wasser + 100µl Polyethylenimin (50% in H₂O) einlegen.
- PP-Nahtmaterial in deionisiertem Wasser waschen.
- PP 2 Stunden in 500ml Wasser + 800µl Heparin-Lösung + 5mg Cyanoborhydrid + HCl (eintropfen lassen, bis pH = 3,5) einlegen.
- PP-Nahtmaterial in deionisiertem Wasser waschen.

## Patentansprüche

1. Medizinisches Nahtmaterial mit einem vorzugsweise im wesentlichen zylindrischen Träger (1),
**dadurch gekennzeichnet, daß**
an dessen Oberfläche (2) ein pharmazeutischer Wirkstoff (3) über eine kovalente Bindung (4) dauerhaft gebunden ist.

2. Medizinisches Nahtmaterial nach Anspruch 1, **dadurch gekennzeichnet, daß** es sich bei dem pharmazeutischen Wirkstoff (3) um einen Wirkstoff mit antimikrobiellen Eigenschaften handelt.

3. Medizinisches Nahtmaterial nach Anspruch 1, **dadurch gekennzeichnet, daß** es sich bei dem pharmazeutischen Wirkstoff (3) um einen Wirkstoff mit antiproliferativen Eigenschaften handelt.

4. Medizinisches Nahtmaterial nach Anspruch 1, **dadurch gekennzeichnet, daß** es sich bei dem pharmazeutischen Wirkstoff (3) um einen Wirkstoff mit zellwachstumsfördernden Eigenschaften handelt.

5. Medizinisches Nahtmaterial nach Anspruch 1, **dadurch gekennzeichnet, daß** verschiedene pharmazeutische Wirkstoffe (3) gleichzeitig gebunden werden.

6. Medizinisches Nahtmaterial nach Anspruch 1, **dadurch gekennzeichnet, daß** die kovalente Bindung (4) über einen Spacer (Zwischenmolekül) erfolgt.

7. Medizinisches Nahtmaterial nach Anspruch 1, **dadurch gekennzeichnet, daß** der Träger (1) aus einem Polymer, insbesondere einem Polyamid, einem Polyester oder Polypropylen besteht.

8. Medizinisches Nahtmaterial nach Anspruch 1, **dadurch gekennzeichnet, daß** der Träger (1) aus einem resorbierbaren Polymer, insbesondere einer Polyglykolsäure oder einem Polydioxanon besteht.

9. Medizinisches Nahtmaterial nach Anspruch 1, **dadurch gekennzeichnet, daß** der Träger eine monofile Ausgestaltung hat.

10. Medizinisches Nahtmaterial nach Anspruch 1, **dadurch gekennzeichnet, daß** der Träger eine geflochtene Ausgestaltung hat.
